# FASCICULE DE BREVET EUROPEEN

(11) **EP 1 133 476 B1**
(45) Date de publication et mention de la délivrance du brevet: **02.08.2006**
(21) Numéro de dépôt: 99956122.8
(22) Date de dépôt: 23.11.1999
(51) Int. Cl.: C07D 233/52, C07C 281/18, A61K 31/4168, A61K 31/155, A61P 19/10

(54) **DERIVES D'IMINOGUANIDINES, LEUR PROCEDE DE PREPARATION, LEUR APPLICATION COMME MEDICAMENTS**
IMINOGUANIDINE DERIVATE, IHRE HERSTELLUNG UND VERWENDUNG ALS ARZNEIMITTEL
IMINOGUANIDINE DERIVATIVES, PREPARATION METHOD, USE AS MEDICINES

(30) Priorité: 24.11.1998 FR 9814780
(43) Date de publication de la demande: 19.09.2001
(73) Titulaire: Aventis Pharma S.A., 92160 Antony (FR); GENENTECH, INC., South San Francisco, CA 94080-4990 (US)
(72) Inventeur: CARNIATO, Denis, Résidence Le Samoa Entrée B, F-06800 Cagnes sur Mer (FR); GADEK, Thomas, R., Oakland, CA 94611 (US); GOURVEST, Jean-François, F-77140 Claye-Souilly (FR); KNOLLE, Jochen, D-65830 Kriftel (DE); PEYMAN, Anurschirwan, D-65779 Kelkheim (DE); RUXER, Jean-Marie, F-92130 Issy les Moulineaux (FR); BODARY, Sarah, C., San Bruno, CA 94066 (US)
(74) Mandataire: Rousseau, Pierrick Edouard
(86) Numéro de dépôt international: PCT/FR1999/002880
(87) Numéro de publication internationale: WO 2000/031044

(56) Documents cités:
- EP-A- 0 820 988
- WO-A-97/34865
- WO-A-97/36580

## Description

La présente invention a pour objet de nouveaux dérivés d'iminoguanidines, leur procédé de préparation, leur application comme médicaments et les compositions pharmaceutiques les renfermant.
EP 0820 988 décrit de manière générique une vaste famille de molécule dérivée de la tyrosine et renfermant un groupement imino présentant une activité inhibitrice de la résorption osseuse et antagonistes du récepteur de la vitronectine.

L'invention a pour objet les composés de formule (I') 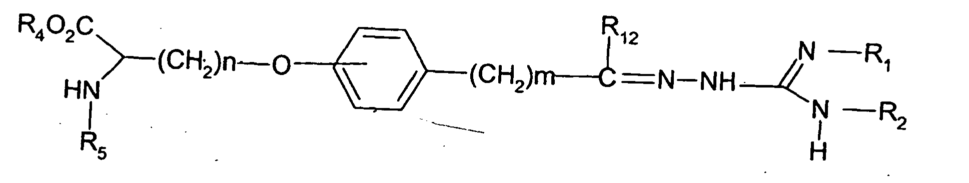 dans laquelle R₁, R₂, R₄, R₅, R₇, R₈ et R₁₂ ont les significations indiquées plus bas, leurs sels physiologiquement acceptables et leurs prodrugs. Les composés de formule (I) sont des composés ayant une activité pharmacologique et sont donc utilisables à titre de médicaments. Ce sont des antagonistes du récepteur de la vitronectine et des inhibiteurs de l'adhésion cellulaire et ils inhibent la résorption osseuse médiée par les ostéoclastes. Ils sont donc utiles pour le traitement thérapeutique ou prophylactique de maladies qui sont causées au moins en partie par une augmentation non désirée de la résorption osseuse, par exemple l'ostéoporose.

L'invention de plus a pour objet les procédés de préparation des composés de formule (I'), leur application, en particulier à titre de médicament et les compositions pharmaceutiques les renfermant.
L'os est constamment soumis à un processus dynamique qui inclut la résorption osseuse et la formation osseuse. Ces processus sont médiés via des cellules spécialisées. La formation osseuse est le résultat du dépôt d'une matrice minérale par les ostéoblastes et la résorption osseuse est le résultat de la dissolution de cette matrice osseuse par les ostéoclastes. La majorité des désordres au niveau de l'os sont basés sur un équilibre perturbé entre la formation osseuse et la résorption osseuse. L'ostéoporose est caractérisée par une perte sèche de cette matrice osseuse. Un ostéoclaste mature activé résorbe l'os après adhésion à la matrice osseuse via la sécrétion d'enzyme protéolytique, et de protons à l'intérieur de la zone d'adhésion, aboutissant à des dépressions ou des creusements de la surface de l'os qui apparaissent au moment où l'ostéoclaste se détache de l'os. Des études ont montré que la fixation de l'ostéoclaste sur l'os est médiée par des récepteurs : les intégrines. Les intégrines sont une superfamille de récepteurs médiant les processus d'adhésion cellule/cellule et plus particulièrement cellule/matrice, incluant notamment α_{IIb}β₃ comme récepteur des plaquettes sanguines (fibrinogène) et αᵥβ₃ comme récepteur de la vitronectine.

Les peptides contenant le motif RGD ainsi que les anticorps anti αᵥβ₃ sont connus pour leur capacité d'inhibition de la résorption de la dentine, d'empêchement de l'adhésion des ostéoclastes sur les matrices minéralisées (Horton et al. Exp. Cell. Res. (1991), 195, 368). Le peptide Echistatine isolé du venin de serpent contenant également un motif RGD et décrit comme inhibiteur de l'adhésion des ostéoclastes à l'os, est un puissant inhibiteur de la résorption osseuse dans les tissus en culture in vitro (Sato et al. J. Cell. Biol. (1990), 111, 1713) et in vivo chez le rat (Fisher et al. Endocrinology (1993) 132, 1411).

Le récepteur αᵥβ₃ est une glycoprotéine transmembranaire qui est exprimée dans un grand nombre de cellules incluant les cellules endothéliales, les cellules du muscle lisse, l'ostéoclaste et des cellules cancéreuses ce qui entraîne ainsi une pluripotentialité des composés de formule (I) selon l'invention.

En effet, les récepteurs αᵥβ₃, exprimés au niveau de la membrane des ostéoclastes, sont à la base du processus d'adhésion/résorption, contribuent à l'organisation du cytosquelette cellulaire et sont impliqués dans l'ostéoporose. Les récepteurs αᵥβ₃ exprimés au niveau des cellules du muscle lisse de l'aorte, stimulent leur migration vers la néointima, ce qui entraîne la formation de l'artériosclérose et la survenue de resténose post-angioplastique (Brown et al., cardiovascular Res. (1994), 28, 1815).

Les cellules endothéliales secrètent des facteurs de croissance qui sont mitogènes pour l'endothélium et peuvent contribuer à la formation de nouveaux vaisseaux sanguins (Angiogénèse).

Les antagonistes de l'intégrine αᵥβ₃ peuvent ainsi entraîner une régression des tumeurs cancéreuses en induisant l'apoptose des vaisseaux sanguins angiogéniques. (Brook et al. Cell (1994) 79, 1157).

Cheresh et al (Science 1995, 270, 1500) ont décrit des anticorps anti-αᵥβ₃ ou des antagonistes du récepteur αᵥβ₃ qui inhibent le processus d'angiogénèse induit par bFGF dans l'oeil de rat, une propriété qui peut être utilisée pour le traitement des rétinopathies, notamment du diabétique.

La demande de brevet WO-A-94/12181 décrit des systèmes aromatiques ou non aromatiques substitués et WO-A-94/08577 décrit des hétérocycles substitués comme antagonistes du récepteur de fibrinogène et inhibiteurs de l'agrégation plaquettaire. EP-A-528 586 et EP-A-528587 décrivent des dérivés de la phénylalanine substitués par un aminoalkyle ou un hétérocycle et WO-A-95/32710 décrivent des dérivés aryliques comme inhibiteurs de la résorption osseuse par les ostéoclastes. WO-A-96/00574 décrit des benzodiazépines et WO-A-96/00730 décrit des composés inhibiteurs du récepteur fibrinogène, en particulier, benzodiazépines qui sont liés à un cycle à 5 chaînons azoté comme antagonistes du récepteur de la vitronectine. DE-A-19654483 décrit des antagonistes du récepteur de la vitronectine dérivés de la tyrosine. DE-A 19629816.4 revendique des dérivés de cycloalkyles comme antagonistes du récepteur de la vitronectine. D'autres investigations ont permis de montrer que les dérivés d'iminoguanidine de formule (I') présentent une forte activité comme inhibiteur du récepteur de la vitronectine et de la résorption osseuse médiée via les ostéoclastes.

L'invention a pour objet les composés de formule (I') 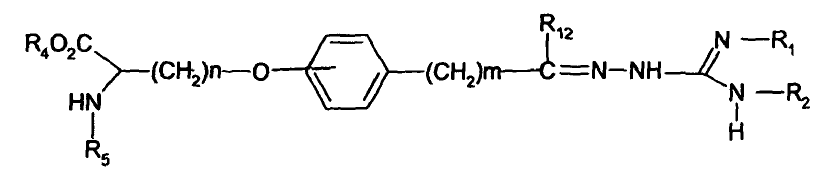 dans laquelle,
R₅ est un radical CO₂R₆, R₆ étant -CH₂Ph, lesdits composés de formule (I) étant sous toutes leurs formes enantiomères, diastéréoisomères et/ou stéréoisomères possibles possibles, seuls ou en mélange selon un rapport quelconque, le groupement (alkyl)iminoguanidine adjacent au phényle étant en position para ou méta de l'oxygène,
   dans laquelle, R₁ et R₂ représentent un atome d'hydrogène ou forment ensemble un groupe (CH₂)ₚ-, dans lequel p est 2 ou 3, ledit radical alkylène ou groupe -(CH₂)ₚ- étant non substitué R₄ représente un atome d'hydrogène ou un groupement (C₁-C₆)-alkyle
R₁₂ représente un atome d'hydrogène ou un radical (C₁-C₆)-alkyle
m est un entier égal à 1 ou 2 ;
n est un entier égal à 2 ;
ainsi que leurs sels physiologiquement acceptables.

Tous les radicaux qui peuvent se retrouver plusieurs fois dans les composés de formule (I') sont indépendants les uns des autres et peuvent être identiques ou différents.

Les radicaux alkyles peuvent être linéaires ou ramifiés, saturés ou mono- ou poly-insaturés. Ceci s'applique également lorsqu'ils portent un substituant ou lorsqu'ils représentent un substituant d'autres radicaux par exemple alkoxy, alkoxycarbonyle, aralkyle ou hétéroarylalkyle.

Par (C₁-C₈)-alkyle on entend les radicaux méthyle, éthyle, propyle, butyle, pentyle, hexyle, heptyle, octyle, nonyle, , les n-isomères de ces radicaux, isopropyle, isobutyle, isopentyle, néopentyle, isohexyle, 3-méthylpentyle, 2,3,4-triméthylhexyle, sec-butyle, tert-butyle, tert-pentyle. Parmi les radicaux préférés, on peut citer méthyle, éthyle, n-propyle, isopropyle, n-butyle, isobutyle, et tert-butyle.

Les radicaux alkylènes bivalents correspondant aux radicaux monovalents cités plus haut sont par exemple les radicaux méthylène, éthylène, 1,3-propylène, 1,2-propylène (=1-méthyléthylène), 2,3-butylène (=1,2-diméthyléthylène), 1,4-butylène, ou 1,6-hexylène.

Les atomes de carbone optiquement actifs contenus dans les composés de formule (I') peuvent indépendamment les uns des autres présenter la configuration R ou la configuration S.

Les composés de formule (I') peuvent être sous la forme d'énantiomères purs ou de diastéréoiscmères purs ou sous la forme d'un mélange d'énantiomères, par exemple sous forme de racémates ou de mélanges de diastéréoisomères.

La présente invention a donc pour objet les énantiomères purs, les mélanges de ces énantiomères, les diastéréoisomères purs et les mélanges de ces diastéréoisomères.

L'invention comprend les mélanges de deux ou plus de deux stéréoisomères de formule (I') et tous les rapports de ces stéréoisomères au sein desdits mélanges.
Les composés de formule (I') peuvent le cas échéant être présent sous forme d'isomères E ou d'isomères Z.

L'invention a donc pour objet les isomères E purs, les isomères Z purs et les mélanges E/Z dans un rapport quelconque.

L'invention comprend également toutes les formes tautomères des composés de formule (I'), par exemple en ce qui concerne la forme représentée par la formule (I'), on considère la forme dans laquelle l'iminoguanidine est présente sous la forme d'un groupe -N-N=C(NHR₁)(NR₂R₇), et toutes les autres formes qui diffèrent par la position différente de l'atome d'hydrogène.

Enfin l'invention comprend les différents régioisomères liés à la position para ou méta de l'oxygène adjacent au phényle. Elle inclut donc les isomères suivants : Oxygène en position 4 et R₈ en position 3, Oxygène en position 4 et R₈ en position 2, Oxygène en position 3 et R₈ en position 4, Oxygène en position 3 et R₈ en position 2, Oxygène en position 3 et R₈ en position 5, oxygène en position 3 et R₈ en position 6. De préférence l'oxygène est en position 4 et R₈ est en position 3.
Les diastéréoisomères, incluant les isomères E/Z peuvent être séparés en isomères individuels, par exemple par chromatographie. Les racémates peuvent être séparés en deux énantiomères par des méthodes courantes telles que la chromatographie en phase chirale ou par des méthodes de résolution.

Les sels physiologiquement acceptables des composés de formule (I') sont en particulier des sels pharmaceutiquement utilisables ou non toxiques ou physiologiquement utilisables.

Lorsque les composés de formule (I') renferment un groupe acide tel que carboxylique, il s'agit par exemple des sels de métaux alcalins ou alcalino terreux tels que les sels de sodium, de potassium, de magnésium, de calcium, et également les sels formés avec des ions ammonium quaternaires physiologiquement acceptables et les sels d'addition avec les acides tel que l'ammoniac et des amines organiques physiologiquement acceptables telles que par exemple la triéthylamine, l'éthanolamine ou le tris-(2-hydroxyethyl)amine.

Lorsque les composés de formule (I') contiennent un groupe basique, ils peuvent former un sel d'addition avec les acides par exemple avec les acides inorganiques tels que l'acide chlorhydrique, sulfurique, phosphorique ou avec les acides organiques carboxyliques tels que l'acide acétique, trifluoroacétique, citrique, benzoique, maléique, fumarique, tartarique, méthanesulfonique ou para toluène sulfonique.

Les composés de formule (I') qui comportent un groupe basique et un groupe acide, tel que par exemple guanidino et carboxylique, peuvent être présents sous forme de Zwiterions (bétaînes), qui sont également inclus dans la présente invention.
L'anion Q- physiologiquement acceptable qui est contenu dans les composés de formule (I') quand R₄ est un radical alkyle substitué par un groupement ammonium chargé, est de préférence un anion monovalent ou un équivalent d'un anion polyvalent d'un acide organique ou inorganique non toxique, physiologiquement acceptable et en particulier pharmaceutiquement acceptable, par exemple l'anion ou un équivalent d'un anion d'un des acides cités plus haut utiles pour la formation des sels d'addition. Q- peut être par exemple un des anions (ou équivalent d'anion) d'un groupe choisi parmi chlore, sulfate, phosphate, acétate, trifluoroacétate, citrate, benzoate, maléate, fumarate, tartrate, méthanesulfonate et para-toluènesulfonate.

Les sels des composés de formule (I') peuvent être obtenus par les méthodes ordinaires connues de l'homme du métier, par exemple en combinant un composé de formule (I') avec un acide organique ou inorganique ou une base dans un solvant ou un dispersant ou à partir d'un autre sel par échange de cation ou d'anion.

L'invention inclut également tous les sels des composés de formule (I') qui, à cause de leur faible acceptabilité physiologique, ne sont pas directement utilisable comme médicament, mais sont utilisables comme produits intermédiaires pour mettre en oeuvre des modifications chimiques ultérieures au niveau des composés de formule (I') ou comme produits de départ pour la préparation de sels physiologiquement acceptables.

La présente invention inclut également tous les solvates des composés de formule (I') par exemples les hydrates, les solvates formé avec les alcools, et tous les dérivés des composés de formule (I'), par exemple les esters, prodrugs et autres dérivés physiologiquement acceptables, ainsi que les métabolites des composés de formule (I').
Parmi les composés préférés de formule (I'), se trouvent les composés dans lesquels le carbone asymétrique portant les groupements CO₂R₄ et NHR₅ est de configuration S.

L'invention a également pour objet les composés de formule (I') dont les noms suivent :
- O-[4-[3-[(4,5-dihydro-1H-imidazol-2-yl)hydrazono]butyl]-phényl]-N-[(phénylméthoxy)carbonyl]-homosérine,
- O-[4-[3-[(aminoiminométhyl)hydrazono]butyl]phényl]-N-[(phénylméthoxy)carbonyl]-homosérine,
- O-[4-[3-[(4,5-dihydro-1H-imidazol-2-yl)hydrazono]propyl]-phényl]-N-[(phénylméthoxy)carbonyl]-homosérine,
- O-[4-[3-[(aminoiminométhyl)hydrazono]propyl]phényl]-N-[(phénylméthoxy)carbonyl]-homosérine,
- O-[4-[2-[(4, 5-dihydro-1H-imidazol-2-yl) hydrazono]propyl]-phényl]-N-[(phénylméthoxy)carbonyl]-homosérine,
- O-[4-[2-[(4,5-dihydro-1H-imidazol-2-yl)hydrazono]propyl]-2-mêthoxyphényl]-N-[(phénylméthoxy)carbonyl]-homosérine,
- O-[4-[2-[(4,5-dihydro-1H-imidazol-2-yl)hydrazono]propyl]-2-fluorophényl]-N-[(phénylméthoxy)carbonyl]-homosérine,
- O-[3-[3-[(aminoiminométhyl)hydrazono]butyl]phényl]-N-[(phénylméthoxy)carbonyl]-homosérine,
- O-[3-[3-[(4,5-dihydro-1H-imidazol-2-yl)hydrazono]butyl]-phényl]-N-[(phénylméthoxy)carbonyl]-homosérine,
- O-[3-[2-[(4,5-dihydro-1H-imidazol-2-yl)hydrazono]propyl]-phényl]-N-[(phénylméthoxy)carbonyl]-homosérine,
- O-[3-[3-[(aminoiminométhyl)hydrazono]propyl]phényl]-N-[(phénylméthoxy)carbonyl]-homosérine,
- O-[3-[3-[(4,5-dihydro-1H-imidazol-2-yl)hydrazono]propyl]-phényl]-N-[(phénylméthoxy)carbonyl]-homosérine,
- O-[4-[(4,5-dihydro-1H-imidazol-2-yl)hydrazono]méthyl]-phényl]-N-[(phénylméthoxy)carbonyl]-homosérine,
ainsi que leurs sels physiologiquement acceptables.

La présente invention a également pour objet un procédé de préparation des composés de formule (I'). Les composés peuvent généralement être préparés, par exemple au cours d'une synthèse convergente par couplage de deux ou plusieurs fragments qui peuvent être dérivés par rétrosynthèse des composés de formule (I). Afin d'éviter que les groupes fonctionnels puissent mener à des réactions indésirables ou secondaires au cours de chaque étape de synthèse, il peut être avantageux ou nécessaire au cours de la synthèse d'introduire les groupes fonctionnels sous forme de précurseurs qui sont par la suite convertis en groupes fonctionnels désirés ou de bloquer temporairement des groupes fonctionnels en mettant en oeuvre une stratégie de groupe protecteur appropriée à la synthèse qui est connue par l'homme de l'art (Greene, Wuts protective Group in Organic Synthesis, Wiley 1991).
Ainsi les composés de formule (I') peuvent être préparés, par exemple, en couplant d'une manière connue en soi un acyle ou formyle de formule (III) 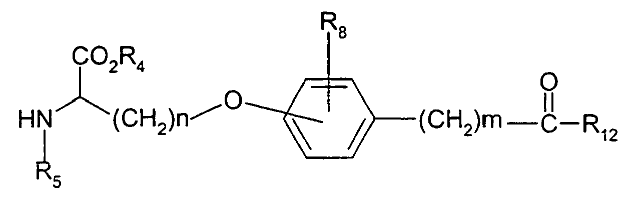 dans laquelle R₄, R₅, R₈, R₁₂, n et m sont tels que définis plus haut pour la formule (I), et où, le cas échéant, les groupes fonctionnels sont sous forme de précurseurs ou sous forme protégée, avec une aminoguanidine ou un dérivé de l'aminoguanidine de formule (IV) 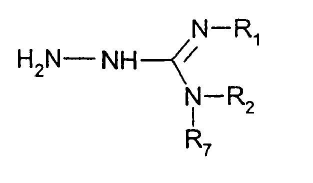 dans laquelle R₁, R₂ et R₇ sont tels que définis dans la formule (I), et où, le cas échéant, les groupes fonctionnels sont sous forme de précurseur ou sous forme protégée, lesdits groupes fonctionnels éventuellement présents sous forme de précurseurs ou sous forme protégée étant par la suite convertis en groupes présents dans les composés de formule (I').

Outre les aminoguanidine libres de formule (IV), les sels d'amino guanidine peuvent également être mis en oeuvre dans la réaction avec les composés de formule (III), les aminoguanidine libres étant formées insitu ou par une étape séparée au moyen d'une base.

La réaction d'un dérivé de formule (III) avec l'amino guanidine (ou dérivé) de formule (IV) est effectuée d'une manière connue en soi, soit sans solvant soit dans un solvant organique protique mais inerte. Dans ce cas on utilise des solvants tels que le l'éthanol ou le butanol.
Le mélange réactionnel est ensuite traité et si désiré le produit réactionnel est purifié selon les méthodes connues de l'homme du métier.

Les groupes protecteurs éventuellement présents dans les composés obtenus à partir des composés de formule (III) et (IV) sont ensuite éliminés par des procédés classiques, par exemple les groupes tert-butyl ester sont convertis en acide carboxylique par traitement avec de l'acide trifluoroacétique, les groupes benzyles sont éliminés par hydrogénation ou encore les groupes fluorénylméthoxycarbonyle sont éliminés en présence d'amine secondaire et d'autres réactions sont mises en oeuvre par des procédés standards, par exemple par des réactions d'acylation. Si nécessaire, la conversion en des sels physiologiquement acceptables s'effectue par des procédés connus de l'homme du métier.

Lorsque R₅ représente un atome d'hydrogène, la fonctionnalisation de l'amine en groupe présent dans les composés de formule (I'), c'est à dire en particulier lorsque R₅ représente un groupement CO₂R₆, s'effectue au niveau des composés de formules (III) ou (I') et de préférence (III). A titre d'exemple pour obtenir les composés de formule (III) avec R₅=CO₂R₆ à partir de l'amine correspondante on fait réagir un composé de formule X'-CO₂R₆, X' étant un groupe partant et notamment O-succinique ou encore un halogène. Pour obtenir les composés de formule (III) avec R₅=SO₂R₆ à partir de l'amine correspondante on fait réagir un composé de formule R₆SO₂X', X' étant notamment un halogène.

Les composés de départs de formule (III) et (IV) qui sont ensuite liés pour donner les composés de formule (I) sont commerciaux, peuvent être préparés selon des procédés décrits dans la littérature ou encore sont accessibles par analogie.
La préparation des composés de formule (III) est illustrée dans le schéma décrit plus bas, étant entendu que la présente invention n'est pas restreinte à ces synthèses ou ces produits de départ. Il n'y a pas de difficulté majeure pour l'homme du métier de prévoir des modifications des synthèses décrites dans notre demande pour la préparation d'autres composés de formule (I) selon l'invention.
Ainsi le composé de formule (V) qui est commercial peut être condensé avec un dérivé halogéné de formule (VI) (décrit dans Arch. Pharm. (1995) 328, 367) pour donner un composé de formule (VII). Cette condensation peut être effectuée par exemple en présence d'une base telle que le carbonate de césium dans l'acétonitrile ou tout autre milieu favorisant les substitutions nucléophiles connues de l'homme du métier.

Le composé de formule (VII) est un exemple de composé de formule (III) dans laquelle R₁₂ est un méthyle.

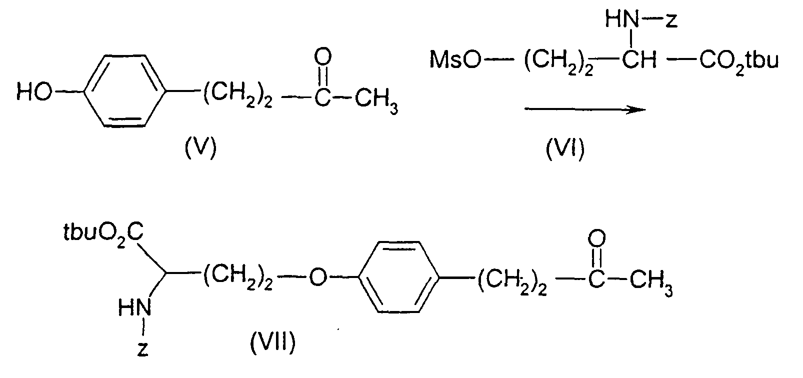

Les composés de formule (I') sont des composés ayant une activité pharmacologique et peuvent ainsi être utilisés à titre de médicaments dans le traitement ou la prévention des maladies de l'os, des maladies tumorales ainsi que des désordres cardiovasculaires.

Les composés de formule (I') ainsi que leurs sels physiologiquement acceptables peuvent être administrés aux animaux, de préférence aux mammifères et en particulier aux êtres humains comme médicaments à titre thérapeutique ou prophylactique. Ils peuvent être administrés tels quels ou en mélange avec un ou plusieurs autres composés de formule (I') ou encore sous la forme d'une préparation pharmaceutique (composition pharmaceutique) qui permet une administration entérale ou parentérale et qui, renferme à titre de composé actif une dose efficace d'au moins un composé de formule (I') et/ou ses sels physiologiquement acceptables ainsi que des supports et/ou additifs courants et pharmaceutiquement inertes.

La présente invention a donc pour objet les composés de formule (I') et/ou leurs sels physiologiquement acceptables à titre de médicament.

La présente invention a également pour objet l'utilisation des composés de formule (I') et/ou leurs sels physiologiquement acceptables pour la préparation de médicaments destinés à la prévention ou au traitement des maladies citées plus haut ou plus bas, par exemple pour le traitement ou la prévention des maladies de l'os.

La présente invention a également pour objet les compositions pharmaceutiques qui permettent une administration entérale ou parentérale et qui, renferment à titre de composé actif une dose efficace d'au moins un composé de formule (I') et/ou ses sels physiologiquement acceptables ainsi qu'un ou plusieurs supports usuels pharmaceutiquement inertes et le cas échéant un ou plusieurs additifs usuels.

Les médicaments peuvent être administrés oralement, par exemple sous forme de pilules, de comprimés, de comprimés enrobés, de pelliculés, de granules, de gélules et capsules molles, de solutions, de sirops, d'émulsion, de suspension ou de mélange d'aérosol.

L'administration peut cependant être effectuée par voie rectale, par exemple sous forme de suppositoire ou par voie parentérale, par exemple sous forme de solutions injectables, d'infusions, de microcapsules ou d'implants ou par voie percutanée, par exemple sous la forme de pommade, de solutions, de pigments ou de colorants ou par d'autre voie telle que sous la forme d'aérosol ou de spray nasal.

Les compositions pharmaceutiques selon l'invention sont préparées selon des méthodes connues en soi, des supports organiques ou inorganiques, pharmaceutiquement inertes étant additionnés aux composés de formule (I') et/ou leurs sels physiologiquement acceptables. Pour la production de pilules, de comprimés, de comprimés enrobés et de capsule en gélatine dure, il est possible d'utiliser par exemple, du lactose, de l'amidon de maïs ou ses dérivés, du talc, de l'acide stéarique ou ses sels, etc. Les supports convenables pour des capsules en gélatine molle ou pour les suppositoires sont par exemple les graisses, les cires les polyols semi-solides ou liquides, les huiles naturelles ou modifiées etc. Les véhicules appropriés pour la préparation de solutions, par exemple les solutions injectables, les émulsions ou les sirops sont par exemple l'eau, les alcools, le glycérol, les polyols, le sucrose, les sucres invertis, le glucose, les huiles végétales, etc.

Les supports convenables pour les microcapsules ou les implants sont par exemple les copolymères d'acide glyoxilique et d'acide lactique. Les préparations pharmaceutiques contiennent normalement de 0,5% à 90% en poids de composés de formule (I) et/ou leurs sels physiologiquement acceptables. En plus des principes actifs et des supports, les préparations pharmaceutiques peuvent contenir des additifs tels que par exemple des diluants, des désintégrants, des liants, des lubrifiants, des agents mouillant, des stabilisants, des émulsifiants, des préservateurs, des agents sucrant, des colorants des agents de flaveurs ou des aromatisants, des épaississants, des agents tampons, et aussi des solvants ou des solubilisants ou des agents pour obtenir un effet retard et également des sels pour modifier la pression osmotique, des agents d'enrobage Ou des antioxydants.

Elles peuvent également contenir deux ou plusieurs composés de formule (I') et/ou leurs sels physiologiquement acceptables. En outre, en plus d'au moins un ou plusieurs composés de formule (I') et/ou leurs sels physiologiquement acceptables, ils peuvent contenir au moins un ou plusieurs autres principes actifs utilisables à titre thérapeutique ou prophylactique. Les préparations pharmaceutiques (compositions pharmaceutiques) renferment normalement de 0,2 à 500 mg, et de préférence de 1 à 200 mg de composé de formule (I') et/ou leurs sels physiologiquement acceptables

Les composés de formule (I') sont tout particulièrement des antagonistes des récepteurs de la vitronectine et sont capables ainsi par exemple d'inhiber l'adhésion des ostéoclastes sur la surface de l'os et ainsi la résorption osseuse par les ostéoclastes.
L'action des composés de formule (I') peut être démontrée par exemple dans un test dans lequel l'inhibition de la liaison de la vitronectine aux cellules qui contiennent le récepteur de la vitronectine est déterminée. Des précisions sur ce test sont données plus bas.

Comme antagonistes du récepteur de la vitronectine, les composés de formule (I') et leurs sels physiologiquement acceptables et leurs prodrugs conviennent en général pour le traitement ou la prévention de maladies liées aux interactions entre les récepteurs de la vitronectine et leurs ligands, dans les processus d'interaction cellule-cellule ou cellule-matrice ou qui peuvent être influencés par l'inhibition des interactions de ce type, pour soulager ou guérir lorsqu'une inhibition des interactions de ce type est désirée.
Comme on l'a expliqué au début, une telle interaction joue un rôle important dans la résorption osseuse, dans l'angiogénèse ou dans la prolifération des cellules du muscle vasculaire smooth.

Les maladies de l'os dont le traitement ou la prévention nécessitent l'emploi des composés de formule (I'), sont notamment l'ostéoporose, l'hypercalcémie, l'ostéopénie, par exemple causée par les métastases osseuses, les désordres dentaires par exemple les parodontites, l'hyperparathyroïdisme, les érosions périarticulaires dans l'arthrite rhumatoïde, et la maladie de Paget. En outre les composés de formule (I') peuvent être utilisés pour soulager, empêcher ou traiter les désordres de l'os qui sont causés par les traitements, par les glucocorticoides, les thérapies liées à la prise de stéroides ou de corticostéroïdes ou par les déficiences d'hormones sexuelles mâles ou femelles.
Tous ces désordres sont caractérisés par une perte osseuse, qui est basée par un défaut d'équilibre entre la formation osseuse et la destruction osseuse et qui peut être influencé favorablement par l'inhibition de la résorption osseuse par les ostéoclastes.

A côté de cette utilisation comme inhibiteur de la résorption osseuse médiée via les ostéoclastes, les composés de formule (I') et leurs sels physiologiquement acceptables et leurs prodrugs sont utilisés comme inhibiteurs de la croissance tumorale ou des métastases cancéreuses, dans le traitement des désordres inflammatoires, pour la thérapie ou la prophylaxie des désordres cardiovasculaires, tels que l'artériosclérose ou la resténose ou la thérapie ou la prophylaxie de néphropatie ou rétinopathie telles que par exemple la rétinopathie diabétique.

Les composés selon l'invention peuvent posséder également une activité vis-à-vis d'autres intégrines qui interagissent avec leur ligand via la séquence tripeptidique RGD (αᵥβ₁, αᵥβ₅, α_{IIb}β₃), leur confèrent des propriétés pharmacologiques utilisables pour traiter les pathologies associées à ces récepteurs.

Cette activité vis-à-vis des intégrines rend ainsi les composés de formule (I') utilisables dans la prévention ou le traitement de nombreuses maladies telles que celles mentionnées plus haut ou dans la revue de Dermot Cox DN§P 8(4) Mai 1995, 197-205 dont le contenu est intégré dans la présente demande.

Lorsqu'on utilise les composés de formule (I'), les doses peuvent varier à l'intérieur de limites larges et doivent être fixées en fonction de la personne à traiter. Ceci dépend par exemple du composé employé ou de la nature et de la sévérité de la maladie à traiter et si on se trouve dans des conditions graves ou chronique ou si on met en oeuvre un traitement prophylactique.
Dans le cas d'une administration par voie orale, la dose quotidienne varie en général de 0,01 à 100 mg/kg et de préférence de 0,1 à 50 mg/kg, en particulier de 0,1 à 5 mg/kg. Par exemple pour un adulte de 75 kg on pourra envisager une dose quotidienne variant de 0,3 à 0,5 mg/kg.

Dans le cas d'une administration par voie intraveineuse, la dose quotidienne varie approximativement de 0,01 à 100 mg/kg et de préférence de 0,05 à 10 mg/kg.
La dose quotidienne peut être divisée, en particulier dans le cas de l'administration de grande quantité de principe actif, en plusieurs, par exemple 2, 3 ou 4 parts. Le cas échéant, en fonction du comportement individuel, il peut être nécessaire d'administrer les différentes doses de manière croissante ou décroissante.

Mis à part l'utilisation des composés de formule (I') comme médicaments, on peut également envisager leur utilisation comme véhicule ou support de composés actifs afin de transporter ces composés actifs de manière spécifique vers un site d'action (Drug targeting, voir Targeted Drug Delivery, R.C. Juliano, Handbook of Experimental Pharmacology, Vol 100, Ed. Born, G.V.R. et al , Springer Verlag). Les composés actifs qui peuvent être transportés sont en particulier ceux utilisés pour le traitement ou la prévention des maladies citées plus haut.

Les composés de formule (I') et leurs sels peuvent également être employés comme agent de diagnostique, par exemple pour des méthodes in vitro ou comme auxiliaire dans des études biochimiques dans lesquelles on désire bloquer le récepteur de la vitronectine ou influencer les interactions cellules-cellules ou cellules-matrices. Ils peuvent en outre être utilisés comme intermédiaires pour la préparation d'autres composés, en particulier d'autres agents actifs, qui sont accessibles à partir des composés de formule (I), part exemple par modification ou introduction de radicaux ou de groupes fonctionnels.

### Exemples

Les produits ont été identifiés par spectre de masse (MS), infrarouge (IR) et/ou spectre RMN. Les composés, qui ont été purifiés par chromatographie en utilisant un éluant qui contient par exemple de l'acide acétique ou trifluoroacétique, et qui ensuite sont séchés ou dans lesquels lors de la dernière étape de synthèse, par exemple de l'acide trifluoroacétique a été utilisé pour éliminer un groupe protecteur tert-butyl, contiennent parfois, en fonction de la manière dont le produit a été séché, l'acide provenant de l'éluant ou de la dernière étape de synthèse et donc se trouvent partiellement ou complètement sous la forme du sel de l'acide utilisé, par exemple sous la forme d'un sel d'acide acétique ou trifluoroacétique. Ils peuvent également être plus ou moins hydratés.
Abbrévation/noms chimiques : PCC : pyridine chlorochromate ; DMF : diméthylformamide ; APTS : acide p-toluène-sulfonique ; THF : tétrahydrofurane ; MeOH : méthanol ; AcOEt acétate d'éthyle ; TFA : acide trifluoroacétique ; TEA : triéthylamine ; ep. (Épaulement) ; F (fort) ; s (singulet) ; d (doublet) ; t (triplet) ; 1 (large) ; m (multiplet).

### Exemple 1 : O-[4-[3-[(4,5-dihydro-1H-imidazol-2-yl)hydrazono]butyl]-phényl]-N-[(phénylméthoxy)carbonyl]-homosérine.

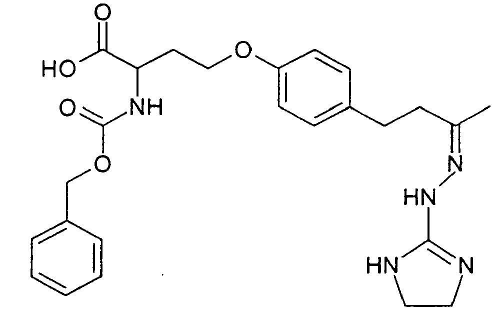

### Stade A : O-[4-(3-oxobutyl)phényl]-N-[(phénylméthoxy) carbonyl]-homoserinate d'éthyle

On chauffe au reflux pendant 1 heure 30 le mélange constitué de 508 mg de 4-(4-hydroxyphényl)-2-butanone (commercial), 1,47 g de 4-bromo-2-[[(phénylméthoxy)carbonyl] amino]butanoate d'éthyle et 2,67 g de carbonate de césium CS₂CO₃ dans 50 ml d'acétonitrile, filtre le CS₂CO₃, lave avec du dichlorométhane puis évapore sous pression réduite jusqu'à obtention de 1,6 g de produit brut que l'on purifie par chromatographie sur une colonne de silice (Kieselgel 60 ; 40-63 µm) en éluant avec le mélange cyclohexane/Acétate d'éthyle 7/3. On obtient 900 mg de produit attendu.
**IR (CHCl**_{**3**}**)**
3429 cm⁻¹ (=C-NH) ; 1717 cm⁻¹ (max. Complexe C=O) ; 1612, 1585, 1512 cm⁻¹ (Aromatique + amide II)

### Stade B : O-[4-[3-[(4,5-dihydro-1H-imidazol-2-yl)hydrazono] butyl-]phényl]-N-[(phénylmêthoxy)carbonyl]-homoserinate d'éthyle

On chauffe à la température de 110°C pendant 2 heures le mélange constitué de 430 mg de la cétone préparée au stade A, 549 mg de bromhydrate de 4,5-dihydro-1H-imidazol-2-yl-hydrazine dans 20 ml de butanol puis évapore sous pression réduite jusqu'à obtention de 1,1 g de produit brut que l'on purifie par chromatographie (Kieselgel 60, 40-63 µm) en éluant avec le mélange dichlorométhane/méthanol 90/10. On obtient 500 mg de produit attendu.
**IR (CHCl**_{**3**}**)**
3446 cm⁻¹ (NH + associé) ; 1738 (ép.), 1720 cm⁻¹ (max.) (C=O) ; 1675 (F), 1605, 1587, 1512 cm⁻¹ (C=N + Aromatique + amide II)

### Stade C : O-[4-[3-[(4,5-dihydro-1H-imidazol-2-yl)hydrazono] butyl]-phényl]-N-[(phénylméthoxy)carbonyl]-homosérine

A une solution de 500 mg d'ester préparé au stade précédent dans 5 ml d'éthanol, on ajoute 1 ml de soude 2N et chauffe au reflux pendant 30 minutes. Après évaporation sous pression réduite, on obtient une huile que l'on reprend avec de l'eau et que l'on neutralise en ajoutant de l'acide chlorhydrique 1N. On évapore une nouvelle fois sous pression réduite et obtient 640 mg d'un produit brut dont une partie (150 mg) est purifiée par chromatographie (Kieselgel 60, 40-65µm) en éluant avec le mélange CH₂Cl₂/méthanol (80/20). On obtient 30 mg de produit attendu sous la forme d'un mélange E/Z 95/5
Rf (CH₂Cl_{2/}MeOH/H₂O/AcOH 88/12/1/1) = 0,25
**IR (Nujol)**
3340, 3245 cm⁻¹ (OH/NH); 1714, 1701, 1662 cm⁻¹ (CO) ; 1611, 1580, 1546, 1513 cm⁻¹ (Système conjugué + Aromatique amide II)
**RMN (CD**_{**3**}**OD)**
1,94 (s) 1,98 (s) CH₃-C=N; 2,03 (m, 1H) 2,34 (m, 1H) CH₂ central ; 2,62 (m, 2H) 2,83 (m, 2H) =C-CH₂-CH₂-C= ; 3,73 (s) 3,78 (s) 4H N-CH₂-CH₂-N ; 4,03 (t, 2H, Ph-O-CH₂-CH₂-) ; 4,23 (m, 1H, -CH(CO₂H)(NHZ)); 5,07 (AB, CO₂CH₂Ph); 6,81 et 7,10 (AA'BB', Ph-O) ; 7,32 (m, 5H aromatique)

### Exemple 2 : O-[4-[3-[(aminoiminométhyl)hydrazono]butyl] phényl]-N-[(phénylméthoxy)carbonyl]-homosérine

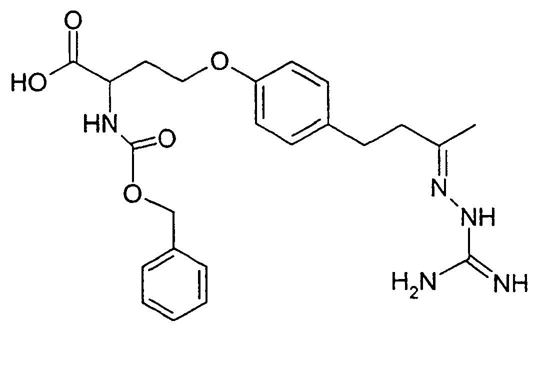

On opère comme à l'exemple 1 stades A, B et C mais en utilisant 440 mg de cétone (obtenue au stade A de l'exemple 1) et 342 mg de chlorhydrate d'aminoguanidine. On obtient 380 mg de produit attendu, après chromatographie (éluant CH₂Cl₂/MeOH/H₂O/AcOH 90/10/1/1) sous forme d'un mélange E/Z 85/15.
**IR (Nujol)**
Absorption région OH/NH ; 1700-1675 cm⁻¹ (C=O) ; 1612, 1585, 1510 cm⁻¹ (C=N + C=O + aromatique + amide II)
RMN (DMSO)
1,92 (s) 2,01 (s) CH₃-C=N; 2,08 (m, 1H) 2,32 (m, 1H) CH₂ central ; 2,62 (t) 2,85 (t) =C-CH₂-CH₂-C= ; 4,02 (t, 2H, Ph-O-CH₂-CH₂-) ; 4,32 (m, -CH(CO₂H) (NHZ)); 5,07 (AB, CO₂CH₂Ph); 6,81 et 7,08 (AA'BB', Ph-O); 7,30 (m, 5H aromatique)

### Exemple 3 : O-[4-[3-[(4,5-dihydro-1H-imidazol-2-yl)hydrazono]propyl]-phényl]-N-[(phénylméthoxy)carbonyl]-homosérine

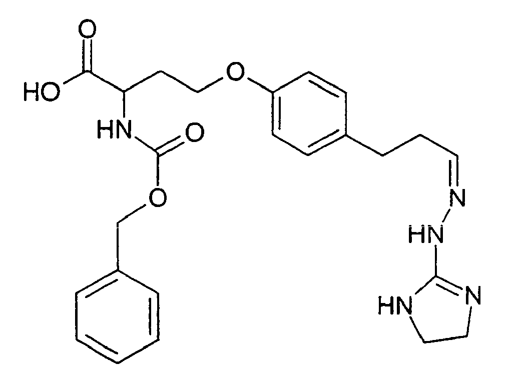

On opère comme à l'exemple 1 stades A, B et C mais en utilisant 190 mg de 3-(4-hydroxyphényl)-propanal et 530 mg de 4-bromo-2-[[(phénylméthoxy)carbonyl]amino]butanoate d'éthyle (Stade A) puis, au cours du stade B, 92 mg de bromhydrate de 4,5-dihydro-1H-imidazol-2-yl-hydrazine. On obtient 20 mg de produit attendu sous forme d'un mélange E/Z 90/10.
**RMN (DMSO)**
1,98 (m, 1H) 2,15 (m, 1H) CH₂ central ; 2,44 (m, 2H) 2,68 (m, 2H) =C-CH₂ et CH₂-Ph ; 3,29 à 3,55 N-CH₂-CH₂-N ; 3,97 (m, Ph-O-CH₂-CH₂- et CH) ; 5,01 (sl, CO₂CH₂Ph) ; 6,60 à 7,20 (aromatique et CH=N) ; 7,34 (sl, aromatique) ; 6,92 (H mobile)

### Exemple 4 : O-[4-[3-[(aminoiminométhyl)hydrazono]propyl] phényl]-N-[(phénylméthoxy)carbonyl]-homosérine

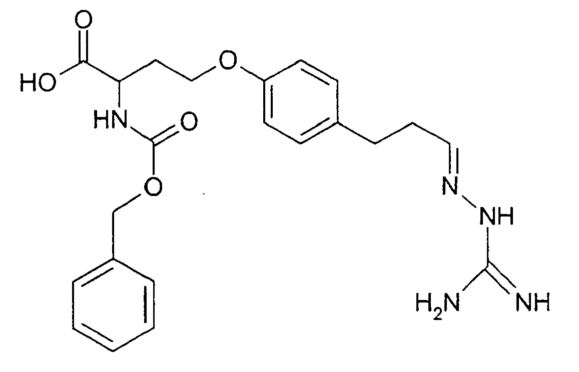

On opère comme à l'exemple 2 stades A, B et C mais en utilisant 190 mg de 3-(4-hydroxyphényl)-propanol et 530 mg de 4-bromo-2-[[(phénylméthoxy)carbonyl]amino]butanoate d'éthyle (Stade A). On obtient 20 mg de produit attendu sous forme de mélange E/Z 75/25.
**RMN (DMSO)**
1,98 (m, 1H) 2,11 (m, 1H) CH₂ central ; 2,43 (m, 2H) 2,65 (m, 2H) =C-CH₂-CH₂-C= ; 3,97 (t, 2H) 3,89 (m, 1H) (Ph-O-CH₂-CH₂- et CH(CO₂H)(NHZ) ; 5,00 (s, CO₂CH₂Ph) ; 6,81 et 7,20, 6,81 et 7,18 (AA'BB' dédoublé aromatique) ; 7,34 (m, aromatique) ; 7,38 (t, en partie masqué =CH-CH₂) ; 7,0 à 8,0 (m, large H mobiles).

### Exemple 5 : O-[4-[2-(4,5-dihydro-1H-imidazol-2-yl)hydrazono] propyl]-phényl]-N-[(phénylméthoxy)carbonyl]-homosérine

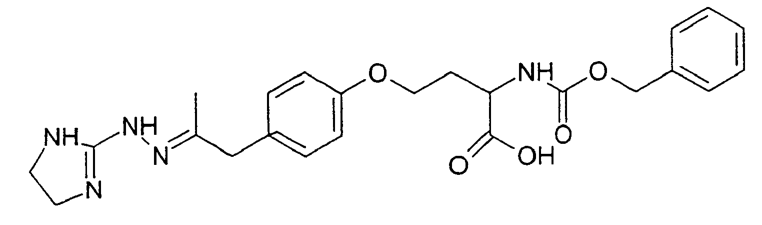

On opère comme à l'exemple 1 stades A, B et C mais en utilisant 800 mg de 3-(4-hydroxyphényl)-2-propanone et 1,83 g de 4-bromo-2-[((phénylméthoxy)carbonyl]amino]butanoate d'éthyle (Stade A) puis, au cours du stade B, 718 mg bromhydrate de 4,5-dihydro-1H-imidazol-2-yl-hydrazine. L'étape de saponificaton s'effectue en présence de soude dans le méthanol. On obtient 180 mg de produit attendu sous forme de mélange E/Z 80/20.
**RMN (DMSO)**
1,72 (s) 1,76 (s) CH₃-C= ; 1,97 (m, 1H) 2,09 (m, 1H) CH₂ central ; 3,42 (s) 3,47 (s) =C-CH₂-C= ; 3,55 (sl) 3,68 (s) N-CH₂-CH₂-N ; 3,88 à 4,05 (m, 3H Ph-O-CH₂-CH₂- + CH(CO₂H)NHZ)); 4,99 (s, CO₂CH₂Ph) ; 6,72 6,79 7,04 7,08 (AA'BB' aromatique) ; 6,92 (dl) 6,58 (m) COCHNH ; 7,33 (m, 5H aromatique) ; 7,74 (m, large H mobile)

### Exemple 6 : O-[4-[2-[(4,5-dihydro-1H-imidazol-2-yl)hydrazono] propyl]-2-méthoxyphényl]-N-[(phénylméthoxy)carbonyl]-homosérine

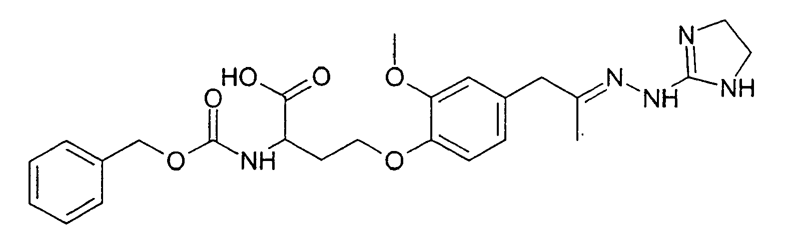

On opère comme à l'exemple 1 stades A, B et C mais en utilisant 900 mg de 3-(4-hydroxy-3-méthoxyphényl)-2-propanone et 1,80 g de 4-bromo-2-[[(phénylméthoxy)carbonyl]amino]butanoate d'éthyle (Stade A) puis, au cours du stade B, 1,47 g de bromhydrate de 4,5-dihydro-1H-imidazol-2-yl-hydrazine. L'étape de saponificaton s'effectue en présence de soude dans le méthanol. On obtient 20 mg de produit attendu sous forme de mélange E/7 90/10
**RMN (DMSO)**
1,90 H₃C-C=N ; 1,90 à 2,10 CH₂ central ; 3,43 2H Ph-CH₂-C=N ; 3,57 =N-CH₂ ; 3,67 (s) Ph-OMe ; 3,97 (m, 3H Ph-O-CH₂-CH₂- + CH(CO₂H)NHZ)) ; 5,00 (sl, CO₂CH₂Ph) ; 6,69 (dd) 6,75 (d, J=1,5) 6,80 (d, J=9) (aromatique) ; 7,07 (d, =C-NH-CH); 7,33 (1, aromatique) ; 7,95 (étalé, H mobile).

### Exemple 7 : O-[4-[2-[(4,5-dihydro-1H-imidazol-2-yl)hydrazono] propyl]-2-fluorophényl]-N-[(phénylméthoxy)carbonyl]-homosérine

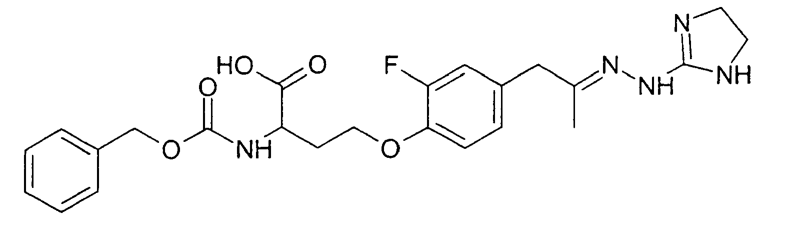

On opère comme à l'exemple 1 stades A, B et C mais en utilisant 515 mg de 3-(3-fluoro-4-hydroxyphényl)-2-propanone et 1,05 g de 4-bromo-2-[[(phénylméthoxy)carbonyl] amino]-butanoate d'éthyle (Stade A) puis, au cours du stade B, 0,517 g de bromhydrate de 4,5-dihydro-1H-imidazol-2-yl-hydrazine. L'étape de saponificaton s'effectue en présence de soude dans le méthanol. On obtient 680 mg de produit attendu sous forme de mélange E/Z 80/20.
**RMN (DMSO)**
1,72 (s) 1,78 (s) CH₃-C= ; 1,99 à 2,11 CH₂ central ; 3,44 (s) 3,50 (s) =C-CH₂-C= ; 3,58 (sl) 3,69 (s) N-CH₂-CH₂-N ; 3,95 (m, CH(CO₂H)NHZ)); 4,07 (t, large) Ph-O-CH₂-CH₂- ; 5,00 (s, CO₂CH₂Ph) ;6,90 à 7,07 (m, 3H aromatique); 7,33 (m, 5H aromatique) ; 7,96 (m) 10,22 (sl) H mobile.

### Exemple 8 : O-[3-[3-[(aminoiminométhyl)hydrazonojbutyl] phényl]-N-[(phénylméthoxy)carbonyl]-homosérine

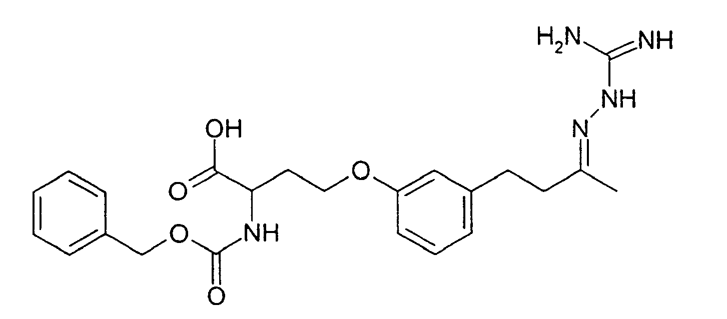

On opère comme à l'exemple 1 stades A, B et C mais en utilisant 390 mg de 4-(3-hydroxyphényl)-2-butanone et 818 mg de 4-bromo-2-[[(phénylméthoxy)carbonyl]amino]butanoate d'éthyle (Stade A). On obtient 180 mg de produit attendu. (PF = 95-100°C) sous forme de mélange E/Z 50/50.
**RMN (DMSO)**
1, 90 (s) 1,91 (s) =C-CH₃ ; 2,05 (m) CH2 central ; 2,55 (m, en partie masqué) 2,79 (t,< 2H) =C-CH₂-CH₂-C= ; 3,98 (m, 3H) (Ph-O-CH₂-CH₂- et CH(CO₂H)(NHZ) ; 5,01 (s, CO₂CH₂Ph) ; 6,69 (m, 2H) 6,80 (dl, 1H) 7,16 (t, 1H) (aromatique) ; 7,34 (m, 5H aromatique) ; 7,74 (m, large H mobiles).

### Exemple 9 : O-[3-[3-[(4,5-dihydro-1H-imidazol-2-yl)hydrazono] butyl]-phényl]-N-[(phénylméthoxy)carbonyl]-homosérine

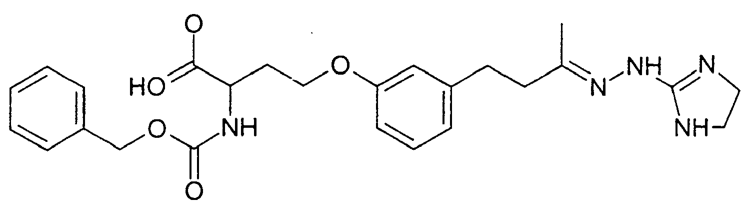

On opère comme à l'exemple 1 stades A, B et C mais en utilisant 390 mg de 4-(3-hydroxyphényl)-2-butanone et 818 g de 4-bromo-2-[[(phénylméthoxy)carbonyl]amino]butanoate d'éthyle (Stade A) puis, au cours du stade B, 380 mg bromhydrate de 4,5-dihydro-1H-imidazol-2-yl-hydrazine. L'étape de saponification s'effectue en présence de soude dans le méthanol. On obtient 110 mg de produit attendu sous forme de mélange E/Z 45/55
**RMN (DMSO)**
1,87 (s) 1,89 (s) (CH₃-C=) ; 2,09 (m, CH₂ central ; 2,78 (m) 2,40 à 2,60 masqué =C-CH₂₋CH₂-C= ; 3, 49 (sl) =N-CH₂- ; 3,80 à 4,10 (3H Ph-O-CH₂-CH₂- + CH(CO₂H)NHZ)) ; 5,01 (s) 5,04 (s), CO₂CH₂Ph) ; 6,66 (sl) H2 ; 6,53 à 6,80 (H5 et H6) ; 6,87 (d) =C-NH-CH ; 7,15 (m, H4) ; 7,35 (m, large 5H, aromatique)

### Exemple 10 : O-[3-[2-[(4,5-dihydro-1H-imidazol-2-yl) hydrazono]propyl]-phényl]-N-[(phénylméthoxy)carbonyl]-homosérine

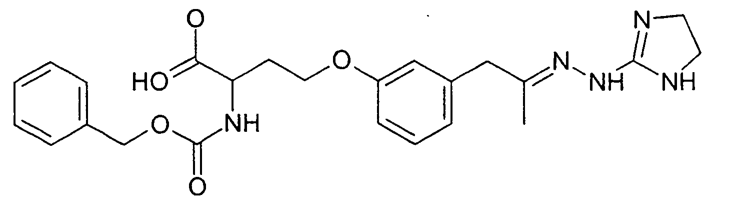

On opère comme à l'exemple 1 stades A, B et C mais en utilisant 100 mg de 4-(3-hydroxyphényl)-2-propanone et 240 mg de 4-bromo-2-[[(phénylmêthoxy)carbonyl]amino]butanoate d'éthyle (Stade A) puis, au cours du stade B, 131 mg de bromhydrate de 4,5-dihydro-1H-imidazol-2-yl-hydrazine. L'étape de saponificaton s'effectue en présence de soude dans le méthanol. On obtient 50 mg de produit attendu sous forme de mélange E/Z
**RMN (DMSO)**
1,71 (s) 1,83 (s) (CH₃-C=) ; 1,95 à 2,15 (m, CH₂ central ; 3,45 (sl) Ph-CH₂C=N- ; 3,53(s), 3,58(s,l) = N-CH₂ ; 3,98 (ml, Ph-O-CH₂ ; + CH(CO₂H)(NHZ)) ; 4,99 (sl, CO₂CH₂Ph) ; 6,63 à 6,76 (3H) 6,89 (sl) 7,07 (t) 7,17 (dd, 1H) H aromatiques ; 7,00 (d, =C-NH-CH)

### Exemple 11 : O-[3-[3-[(aminoiminométhyl)hydrazono]propyl] phényl]-N-[(phénylméthoxy)carbonyl]-homosérine

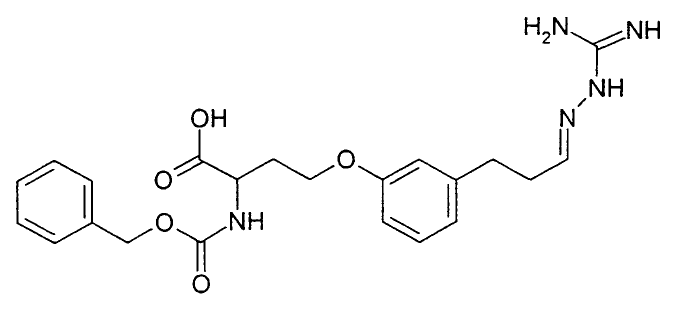

### Stade A: O-[4-(3-hydroxypropyl)phényl]-N-[(phénylméthoxy) carbonyl]-homosérinate d'éthyle.

On chauffe au reflux pendant 45 minutes le mélange constitué de 306 mg de 4-hydroxybenzènepropanol, 770 mg de 4-bromo-2-[[(phénylméthoxy)carbonyl]amino]butanoate d'éthyle et 740 mg de Cs₂CO₃ dans 10 ml d'acétonitrile, filtre le solide, lave avec du dichlorométhane puis évapore sous pression réduite le filtrat jusqu'à obtention de 1,5 g de produit brut que l'on purifie par chromatographie sur une colonne de silice (Kieselgel 60 ; 40-63 µm)en éluant avec le mélange dichlorométhane/méthanol 95/5. On obtient 440 mg de produit attendu.
**IR (CHCl**_{**3**}**)**
3624 cm⁻¹ (-OH) ; 3431 cm⁻¹ (NH) ; 172 cm⁻¹ (max. C=O) ; 1609, 1602, 1584, 1508 cm⁻¹ (Aromatique + amide II)

### Stade B : oxydation O-[4-(3-oxopropyl)phényl]-N-[(phénylméthoxy)carbonyl]-homoserinate d'éthyle

On mélange pendant 30 minutes à température ambiante 245 mg de l'alcool préparé au stade précédent et 254 mg de PCC dans 3 ml de dichlorométhane, puis ajoute 126 mg de PCC supplémentaire et agite 15 minutes. On évapore sous pression réduite jusqu'à obtention de 800 mg de produit brut qui est purifié par chromatographie en éluant avec le mélange acétate d'éthyle/cyclohexane 1/1. On obtient 100 mg de produit attendu.
**IR (CHCl**_{**3**}**)**
3432 cm⁻¹ (NH) ; 1724 cm⁻¹ (max. C=O) ; 1610, 1602, 1585, 1508, 1492 cm⁻¹ (Aromatique + amide II)

### Stade C : condensation O-[3-[3-[(aminoiminométhyl)hydrazono]propyl]phényl]-N-[(phénylméthoxy)carbonyl]-homosérinate d'éthyle

On chauffe à la température de 110°C pendant 15 minutes le mélange constitué de 302 mg de l'aldehyde préparé au stade B, 248 mg de chlorhydrate d'aminoguanidine dans 5 ml de butanol auquel on a ajouté quelques cristaux d'APTS, puis évapore sous pression réduite jusqu'à obtention de 500 mg de produit brut que l'on purifie par chromatographie (Kieselgel 60, 40-63 µm) en éluant avec le mélange dichlorométhane/méthanol 90/10. On obtient 265 mg de produit attendu.
**IR (CHCl**_{**3**}**)**
3466, 3432 cm⁻¹ + associé (région NH/NH2) ; 1736 (ép.), 1721 cm⁻¹ (max.) (C=O) ; 1675 (F, C=N) ; 1636, 1610, 1602, 1585, 1508 , 1489 cm⁻¹ (C=N + Aromatique + NH₂+ amide II)

### Stade D : Saponification O-[3-[3-[(aminoiminométhyl)hydrazono]propyl]phényl]-N-[(phénylméthoxy)carbonyl]-homosérine

A une solution de 255 mg d'ester préparé au stade précédent dans 5 ml d'éthanol, on ajoute 560 µl de soude 2N et chauffe au reflux pendant 15 minutes. Après évaporation sous pression réduite, on obtient 320 mg d'un produit brut que l'on purifie par chromatographie (Kieselgel 60, 40-65 µm) en éluant avec le mélange dichlorométhane/méthanol/eau/acide acétique 90/10/1/1. On obtient 125 mg de produit attendu sous la forme d'un mélange E/Z.
**IR (Nujol)**
3466, 3337 cm⁻¹, Absorption région OH/NH + absorption générale ; 1697 cm⁻¹ (C=O) ; 1675 cm⁻¹ (C=N); 1636, 1609, 1584, 1533, 1487 cm⁻¹ (C=N + Aromatique + amide II)
**RMN (DMSO)**
2,05 (CH₂ central); 2,49 (masqué) 2,78 (m) =C-CH₂-CH₂-C=N; 3,93 (q, CH(CO₂H) (NHZ)) ; 4,06 (m, Ph-O-CH₂) ; 5,02 (sl, CO₂CH₂Ph) ; 6,69 (dt) 6,76 (dl) H4 et H6 ; 6,74 (s1, H2) ; 7,15 (t, H5) ; 7,35 (1, H aromatique et NH) ; 7,48 (t, -N=CH-CH₂).

### Exemple 12 : O-[3-[3-[(4,5-dihydro-1H-imidazol-2-yl) hydrazono]propyl]-phényl]-N-[(phénylméthoxy)carbonyl]-homosérine

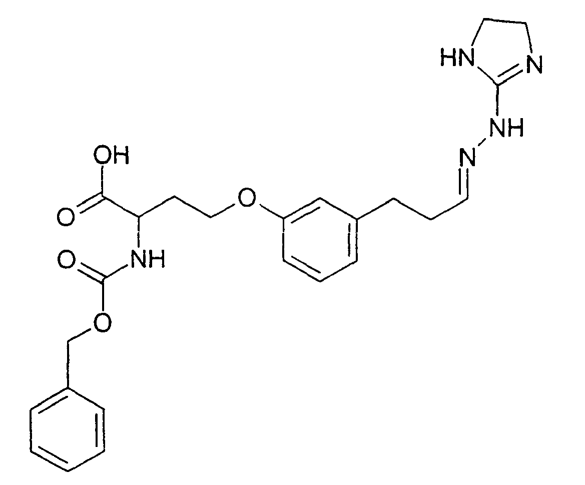

On opère comme à l'exemple 11 stades A, B, C et D mais en utilisant au stade C le bromhydrate de 4,5-dihydro-1H-imidazol-2-yl-hydrazine.
**IR (Nujol)**
Absorption région OH/NH ; 1700 cm⁻¹ (C=O) ; 1675, 1600, 1582, 1486 cm⁻¹ (C=N + Aromatique + amide II)
**RMN (DMSO)**
2,00 à 2,15 (CH₂ central) ; 2,60 à 3,65 8H =C-CH₂ et =N-CH₂ ; 3,92 (1, 1H, CH(CO₂H)(NHZ)) ; 4,05 (m 1, Ph-O-CH₂) ; 5,02 (s) 5,04 (s), CO₂CH₂Ph ; 6,60 à 6,95 (3H) 7,15 (m, 1H) H aromatique ; 7,35 (1, -N=CH-CH₂ et Ph-CH₂).

### Exemple 13 : O-[4-[(4,5-dihydro-1H-imidazol-2-yl)hydrazono] méthyl]phényl]-N-[(phénylméthoxy)carbonyl]-homosérine

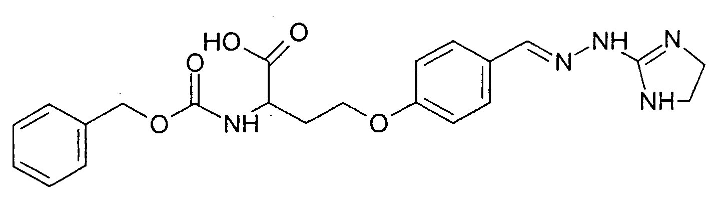

### Stade A : O-[4-(hydroxyméthyl)phényl]-N-[(phénylméthoxy) carbonyl]-homoserinate d'éthyle

On chauffe au reflux pendant 45 minutes le mélange constitué de 690 mg de 4-hydroxybenzènethanol, 1,72 g de 4-bromo-2-[[(phénylméthoxy)carbonyl]amino]butanoate d'éthyle et 1,63 g de Cs₂CO₃ dans 100 ml d'acétonitrile, filtre le solide lave avec du dichlorométhane puis évapore sous pression réduite le filtrat jusqu'à obtention de 2,6 g de produit brut que l'on purifie par chromatographie sur une colonne de silice (Kieselgel 60 ; 40-63 µm) en éluant avec le mélange dichlorométhane/méthanol 95/5. On obtient 1,58 g de produit attendu.
**IR (CHCl**_{**3**}**)**
3618 cm⁻¹ (-OH) ; 3434 cm⁻¹ (NH) ; 1721 cm⁻¹ (max. C=O) ; 1610, 1582, 1512 cm⁻¹ (Aromatique + amide II)

### Stade B : oxydation O-[4-formylphényl]-N-[(phénylméthoxy)carbonyl]-homoserinate d'éthyle

On mélange pendant 1 heure à température ambiante 1,31 g de l'alcool préparé au stade précédent et 1,41 g de PCC dans 30 ml de dichlorométhane, évapore sous pression réduite jusqu'à obtention de 800 mg de produit brut et purifie par chromatographie en éluant avec le mélange acétate d'éthyle/cyclohexane 1/1. On obtient 575 mg de produit correspondant à l'obtention du 4-formylphényle au lieu de 4-formylméthylphényle attendu.
**IR (CHCl**_{**3**}**)**
3424 cm⁻¹ (NH) ; 1721, 1699 cm⁻¹ (C=O) ; 1601,1580,1510 cm⁻¹ (Aromatique + amide II)

### Stade C : condensation O-[4-[(4,5-dihydro-1H-imidazol-2-yl)hydrazono]méthyl]phényl]-N-[(phénylméthoxy)carbonyl]-homoserinate d'éthyle.

On chauffe à la température de 110°C pendant 15 minutes le mélange constitué de 160 mg de l'aldehyde préparé au stade A, 83 mg de bromhydrate de 4,5-dihydro-1H-imidazol-2-yl-hydrazine dans 20 ml de butanol auquel on a ajouté quelques cristaux d'APTS, puis évapore sous pression réduite jusqu'à obtention de 253 mg de produit brut que l'on purifie par chromatographie (Kieselgel 60 , 40-63 µm) en éluant avec le mélange dichlorométhane/méthanol/hydroxyde d'ammonium 90/10/1. On obtient 190 mg de produit attendu.
**IR (CHCl**_{**3**}**)**
3449 cm⁻¹ (NH) ; 1721 cm⁻¹ (C=O) ; 1638, 1608, 1595, 1568, 1511 cm⁻¹ (C=N + Aromatique + amide II)

### Stade D : Saponification O-[4-[(4,5-dihydro-1H-imidazol-2-yl)hydrazono]méthyl]phényl]-N-[(phénylméthoxy)carbonyl]-homosérine

A une solution de 168 mg d'ester préparé au stade précédent dans 20 ml de méthanol, on ajoute 0,36 ml de soude 2N et chauffe au reflux pendant 30 minutes. Après évaporation sous pression réduite, on obtient 170 mg d'un produit brut que l'on purifie par chromatographie (Kieselgel 60, 40-3µm) en éluant avec le mélange dichlorométhane/méthanol/eau/acide acétique 85/15/1/1. On obtient 150 mg de produit attendu.
**Point de fusion = 132°C**
**IR (Nujol)**
Absorption générale OH/NH ; 1670 cm⁻¹ (C=O) ; 1604, 1572, 1510 cm⁻¹ (système conjugué + Aromatique + amide II)
**RMN (DMSO)**
2,02 (m) 2,13 (m) CH₂ central ; 3,47 (sl, 4H N-CH₂-CH₂-N ; 3,93 (m, tl après échange), CH(CO₂H)(NHZ)) ; 6,97 (dl) 7,08 (d) mobile CH(CO₂H)(NHZ) ; 4,04 (t, 2H, Ph-O-CH₂) ; 5,01 (sl, CO₂CH₂Ph) ; 6,88 et 7,57 (AA'BB' phényle) ; 7,34 (m, 5H aromatiques + 1H mobile) ; 7,88 (t, -N=CH-C=).

### Test pharmacologique

### Test ELISA Kistrine/Récepteur Vitronectine (αᵥβ₃)

### Protocole

Des plaques 96 puits MaxiSorp sont coatées une nuit à 40°C avec 100 µl de Kistrine à 1 µg/ml (dilution en tampon de coating : carbonate 0,05M / NaOH pH 9,6). Le lendemain, les puits sont vidés et les ligands (kistrine) sont ensuite fixés (tampons de fixation : PBS contenant 0,5 % de BSA (pH=7,4)) pendant 1 heure à température ambiante avec une agitation douce de 125 rpm. Les puits sont lavés six fois (tampon de lavage :PBS contenant 0,05% de Tween 20 (pH 7,7) puis on ajoute par puits et dans cet ordre :
- 40 µl de tampon d'incubation
- 10 µl de la dilution du produit à tester (les produits sont dilué dans un mélange 50:50 DMSO/Eau)
- 50 µl de récepteur αᵥβ₃ humain (cf Pytel et al. Méthods Enzymol. (1987) 144 (Dilution en tampon d'incubation, à adapter suivant le lot de récepteur et selon le ligand). Le ligand, le récepteur αᵥβ₃ et les produits à étudier sont incubés pendant 3 heures à température ambiante avec une agitation douce de 125rpm.
Les puits sont à nouveau lavés six fois, puis incubés pendant 2 heures à température ambiante avec une agitation douce de 125 rpm, en présence de 100 µl d'anticorps anti-récepteur couplé à une peroxydase (L'anticorps 4B12-HRP est dilué en tampon d'incubation (50 mM TRIS pH 7,4; 0,5% BSA ; 0,05 % Tween 20 ; 1 mM MnCl₂ ; 50 µM CaCl₂ ; 50 µM MgCl₂ ; 100 mM NaCl). La dilution est à adapter suivant le lot de récepteur.

Les puits sont ensuite lavés six fois avant la mesure de la liaison ligand-récepteur faite par l'intermédiaire d'un kit révélateur de peroxydase (TBM Microwell Peroxidase Substrate System Kirkegaard ; Réf cat 50-76-00).

Ce kit contient un flacon A de substrat (3,3',5,5'-tétraméthylbenzidine à 0,4 g/l) et un flacon B (H₂O₂ à 0,02 % en tampon Citrate/Acide citrique). Extemporanément, un volume de A est mélangé à un volume de B, puis le mélange réactionnel est distribué à raison de 100 µl/puits.

La réaction enzymatique se développe entre 6 à 10' pour Kistrine/αᵥβ₃ puis son évolution est stoppée par l'addition de 100 µl d'acide phosphorique 1M. La densité optique est déterminée à 450 nm.

### Expression des résultats

On trace la courbe suivante : le pourcentage de liaison en fonction du logarithme de chaque concentration du produit testé.
Pour chaque produit, on détermine l'IC50 suivant la formule suivante : IC50=(BO + Bmin)/2
BO = Maximum de liaison en l'absence de tout produit
Bmin = Minimum de liaison en présence de la concentration la plus élevée de produit.

| **Exemple** | **K/VnR IC50 (µM)** |
|---|---|
| ex 1 | 0,15 |
| ex 2 | 1,35 |
| ex 3 | 0,11 |
| ex 4 | 0,45 |
| ex 5 | 0,022 |
| ex 6 | 0,012 |
| ex 7 | 0,022 |
| ex 9 | 2 |
| ex 10 | 0,355 |
| ex 11 | 3,25 |
| ex 12 | 0,96 |
| ex 13 | 0,292 |

## Revendications

1. Un composé de formule (I') : 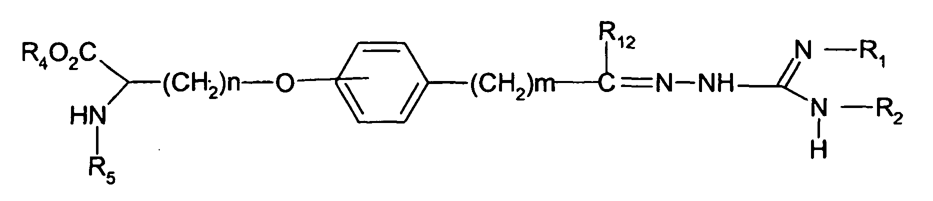 dans laquelle,
R₅ est un radical CO₂R₆, R₆ étant -CH₂Ph, lesdits composés de formule (I) étant sous toutes leurs formes énantiomères, diastéréoisomères et/ou stéréoisomères possibles seuls ou en mélange selon un rapport quelconque, le groupement (alkyl)iminoguanidine adjacent au phényle étant en position para ou méta de l'oxygène,
dans laquelle, R₁ et R₂ représentent un atome d'hydrogène ou forment ensemble un groupe -(CH₂)ₚ-, dans lequel p est 2 ou 3, ledit radical alkylène ou groupe -(CH₂)ₚ- étant non substitué R₄ représente un atome d'hydrogène ou un groupement (C₁-C₆)-alkyle R₁₂ représente un atome d'hydrogène ou un radical (C₁-C₆)-alkyle
m est un entier égal à 1 ou 2 ;
n est un entier égal à 2 ;
ainsi que leurs sels physiologiquement acceptables.

2. Un composé de formule (I') tel que défini à la revendication 1 dont les noms suivent :
- O-[4-[3-[(4, 5-dihydro-1H-imidazol-2-yl)hydrazono]butyl]-phényl]-N-[(phénylméthoxy)carbonyl]-homosérine,
- O-[4-[3-[(aminoiminométhyl)hydrazono]butyl]phényl]-N-[(phénylméthoxy)carbonyl]-homosérine,
- O-[4-[3-[(4,5-dihydro-1H-imidazol-2-yl)hydrazono]propyl]-phényl]-N-[(phénylméthoxy)carbonyl]-homosérine,
- O-[4-[3-[(aminoiminométhyl)hydrazono]propyl]phényl]-N-[(phénylméthoxy)carbonyl]-homosérine,
- O-[4-[2-[(4,5-dihydro-1H-imidazol-2-yl)hydrazono]propyl]-phényl]-N-[(phénylméthoxy)carbonyl]-homosérine,
- O-[4-[2-[(4,5-dihydro-1H-imidazol-2-yl)hydrazono]propyl]-2-méthoxyphényl]-N-[(phénylméthoxy)carbonyl]-homosérine,
- O-[4-[2-[(4,5-dihydro-1H-imidazol-2-yl)hydrazono]propyl]-2-fluorophényl]-N-[(phénylméthoxy)carbonyl]-homosérine,
- O-[3-[3-[(aminoiminométhyl)hydrazono]butyl]phényl]-N-[(phénylméthoxy)carbonyl]-homosérine,
- O-[3-[3-[(4,5-dihydro-1H-imidazol-2-yl)hydrazono]butyl]-phényl]-N-[(phénylméthoxy)carbonyl]-homosérine,
- O-[3-[2-[(4,5-dihydro-1H-imidazol-2-yl)hydrazono]propyl]-phényl]-N-[(phénylméthoxy)carbonyl]-homosérine,
- O-[3-[3-[(aminoiminométhyl)hydrazono]propyl]phényl]-N-[(phénylméthoxy)carbonyl]-homosérine,
- O-[3-[3-[(4,5-dihydro-1H-imidazol-2-yl)hydrazono]propyl]-phényl]-N-[(phénylméthoxy)carbonyl]-homosérine,
- O-[4-[(4,5-dihydro-1H-imidazol-2-yl)hydrazono]méthyl]-phényl]-N-[(phénylméthoxy)carbonyl]-homosérine,
ainsi que leurs sels physiologiquement acceptables.

3. Un procédé de préparation des composés de formule (I') tels que définis à l'une quelconque des revendications 1 à 2, qui comprend le couplage de deux ou plusieurs fragments qui peuvent être dérivés par rétrosynthèse des composés de formule (I').

4. Un procédé selon la revendication 3 dans lequel on fait réagir un acyle ou formyle de formule (III) 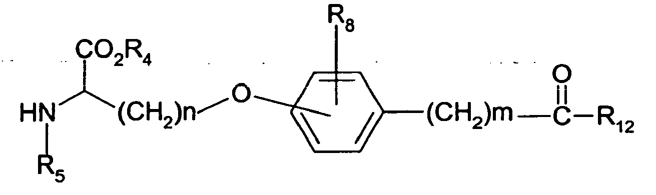 dans laquelle R₄, R₅, R₈, R₁₂, n et m sont tels que définis à la revendication 1, et où, le cas échéant, les groupes fonctionnels sont sous forme de précurseurs ou sous forme protégée, avec une aminoguanidine ou un dérivé de l'aminoguanidine de formule (IV) 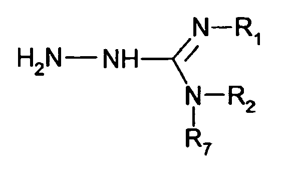 dans laquelle R₁, R₂ et R₇ sont tels que définis à la revendication 1, et où, le cas échéant, les groupes fonctionnels sont sous forme de précurseurs ou sous forme protégée, lesdits groupes fonctionnels éventuellement présents sous forme de précurseurs ou sous forme protégés étant par la suite convertis en groupes présents dans les composés de formule (I').

5. A titre de médicament, un composé de formule (I') et/ou ses sels physiologiquement acceptables tel que défini à l'une quelconque des revendications 1 à 2.

6. Une composition pharmaceutique comprenant au moins un composé de formule (I') tel que défini à l'une quelconque des revendications 1 à 2 et/ou ses sels physiologiquement acceptables ainsi qu'un ou plusieurs supports usuels pharmaceutiquement inertes et le cas échéant un ou plusieurs additifs.

7. A titre de médicament ayant une activité antagoniste du récepteur de la vitronectine, un composé de formule (I') et/ou ses sels physiologiquement acceptables tel que défini à l'une quelconque des revendications 1 à 2.

8. A titre de médicament ayant une activité inhibitrice de la résorption osseuse ou pour le traitement ou la prévention de l'ostéoporose, un composé de formule (I') et/ou ses sels physiologiquement acceptables tel que défini à l'une ... quelconque des revendications 1 à 2.

9. A titre de médicament ayant une activité inhibitrice de la croissance tumorale ou des métastases cancéreuses, un composé de formule (I') et/ou ses sels physiologiquement acceptables tel que défini à l'une quelconque des revendications 1 à 2.

10. A titre de médicament ayant une activité anti-inflammatoire ou pour le traitement ou la prévention des désordres cardiovasculaires, la resténose, l'artériosclérose, les néphropathies ou rétinopathies, un composé de formule (I') et/ou ses sels physiologiquement acceptables tel que défini à l'une quelconque des revendications 1 à 2.

11. Utilisation des composés de formule (I') et/ou leurs sels physiologiquement acceptables tels que définis à l'une quelconque des revendications 1 à 2 pour la préparation de médicaments destinés à la prévention ou au traitement de l'ostéoporose.

12. Utilisation des composés de formule (I') et/ou leurs sels physiologiquement acceptables tels que définis à l'une quelconque des revendications 1 à 2 pour la préparation de médicaments destinés à inhiber la croissance tumorale ou les métastases cancéreuses.

13. Utilisation des composés de formule (I') et/ou leurs sels physiologiquement acceptables tels que définis à l'une quelconque des revendications 1 à 2 pour la préparation de médicaments destinés à la prévention ou au traitement des désordres cardiovasculaires de la resténose, de l'artériosclérose, des néphropathies ou des rétinopathies.

## Claims

1. A compound of formula (I'): 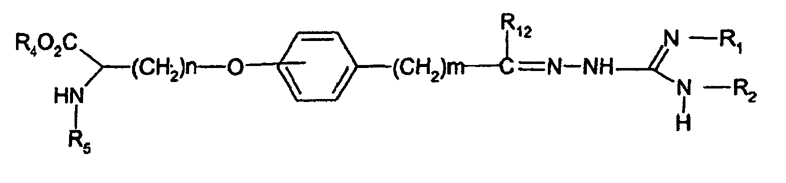 in which,
R₅ is a CO₂R₆ radical, R₆ being -CH₂Ph, the said compounds of formula (I) being in all their possible enantiomeric, diastereoisomeric and/or stereoisomeric forms, alone or as a mixture according to any ratio, the (alkyl)iminoguanidine group adjacent to the phenyl being in the para or meta position with regard to the oxygen,
in which R₁ and R₂ represent a hydrogen atom or together form a -(CH₂)ₚ- group in which p is 2 or 3, the said alkylene radical or -(CH₂)ₚ- group being unsubstituted,
R₄ represents a hydrogen atom or a (C₁-C₆)alkyl group,
R₁₂ represents a hydrogen atom or a (C₁-C₆)alkyl radical, m is an integer equal to 1 or 2;
n is an integer equal to 2;
and its physiologically acceptable salts.

2. A compound of formula (I') as defined in Claim 1, the names of which follow:
- O-[4-[3-[(4,5-dihydro-1H-imidazol-2-yl)hydrazono]-butyl]phenyl]-N-[(phenylmethoxy)carbonyl]homoserine,
- O-[4-[3-[(aminoiminomethyl)hydrazono]butyl]phenyl]-N-[(phenylmethoxy)carbonyl]homoserine,
- O-[4-[3-[(4,5-dihydro-1H-imidazol-2-yl)hydrazono]-propyl]phenyl]-N-[(phenylmethoxy)carbonyl]homoserine,
- O-[4-[3-[(aminoiminomethyl)hydrazono]propyl]phenyl]-N-[(phenylmethoxy)carbonyl]homoserine,
- O-[4-[2-[(4,5-dihydro-1H-imidazol-2-yl)hydrazono]-propyl]phenyl]-N-[(phenylmethoxy)carbonyl]homoserine,
- O-[4-[2-[(4,5-dihydro-1H-imidazol-2-yl)hydrazono]-propyl]-2-methoxyphenyl]-N-[(phenylmethoxy)carbonyl]-homoserine,
- O-[4-[2-[(4,5-dihydro-1H-imidazol-2-yl)hydrazono]-propyl]-2-fluorophenyl]-N-[(phenylmethoxy)carbonyl]-homoserine,
- O-[3-[3-[(aminoiminomethyl)hydrazono]butyl]phenyl]-N-[(phenylmethoxy)carbonyl]homoserine,
- O-[3-[3-[(4,5-dihydro-1H-imidazol-2-yl)hydrazono]-butyl]phenyl]-N-[(phenylmethoxy)carbonyl]homoserine,
- O-[3-[2-[(4,5-dihydro-1H-imidazol-2-yl)hydrazono]-propyl]phenyl]-N-[(phenylmethoxy)carbonyl]homoserine,
- O-[3-[3-[(aminoiminomethyl)hydrazono]propyl]phenyl]-N-[(phenylmethoxy)carbonyl]homoserine,
- O-[3-[3-[(4,5-dihydro-1H-imidazol-2-yl)hydrazono]-propyl]phenyl]-N-[(phenylmethoxy)carbonyl]homoserine,
- O-[4-[[(4,5-dihydro-1H-imidazol-2-yl)hydrazono]-methyl]phenyl]-N-[(phenylmethoxy)carbonyl]homoserine,
and their physiologically acceptable salts.

3. A process for the preparation of the compounds of formula (I') as defined in either one of Claims 1 and 2, which comprises the coupling of two or more fragments which can be derived by retrosynthesis from the compounds of formula (I').

4. A process according to Claim 3, in which an acyl or formyl of formula (III) 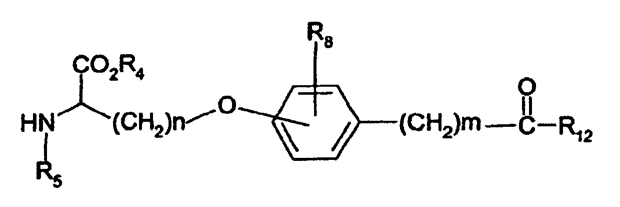 in which R₄, R₅, R₈, R₁₂, n and m are as defined in Claim 1 and where, if appropriate, the functional groups are in the form of precursors or in the protected form, is reacted with an aminoguanidine or a derivative of the aminoguanidine of formula (IV) 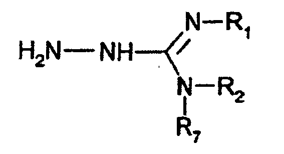 in which R₁, R₂ and R₇ are as defined in Claim 1 and where, if appropriate, the functional groups are in the form of precursors or in the protected form, the said functional groups optionally present in the form of precursors or in the protected form subsequently being converted to groups present in the compounds of formula (I').

5. As medicament, a compound of formula (I') and/or its physiologically acceptable salts as defined in either one of Claims 1 and 2.

6. A pharmaceutical composition comprising at least one compound of formula (I') as defined in any one of Claims 1 and 2 and/or its physiologically acceptable salts, as well as one or more conventional pharmaceutically inert supports and, if appropriate, one or more additives.

7. As medicament having a vitronectin receptor antagonist activity, a compound of formula (I') and/or its physiologically acceptable salts as defined in either one of Claims 1 and 2.

8. As medicament having a bone resorption inhibitory activity or for the treatment or prevention of osteoporosis, a compound of formula (I') and/or its physiologically acceptable salts as defined in either one of Claims 1 and 2.

9. As medicament having a tumour growth or cancer metastasis inhibitory activity, a compound of formula (I') and/or its physiologically acceptable salts as defined in either one of Claims 1 and 2.

10. As medicament having an anti-inflammatory activity or for the treatment or prevention of cardiovascular disorders, restenosis, arteriosclerosis, nephropathies or retinopathies, a compound of formula (I') and/or its physiologically acceptable salts as defined in either one of Claims 1 and 2.

11. Use of the compounds of formula (I') and/or their physiologically acceptable salts as defined in either one of Claims 1 and 2 in the preparation of medicaments intended for the prevention or treatment of osteoporosis.

12. Use of the compounds of formula (I') and/or their physiologically acceptable salts as defined in either one of Claims 1 and 2 in the preparation of medicaments intended to inhibit tumour growth or cancer metastases.

13. Use of the compounds of formula (I') and/or their physiologically acceptable salts as defined in either one of Claims 1 and 2 in the preparation of medicaments intended for the prevention or treatment of cardiovascular disorders, of restenosis, of arteriosclerosis, of nephropathies or of retinopathies.

## Patentansprüche

1. Verbindung der Formel (I') 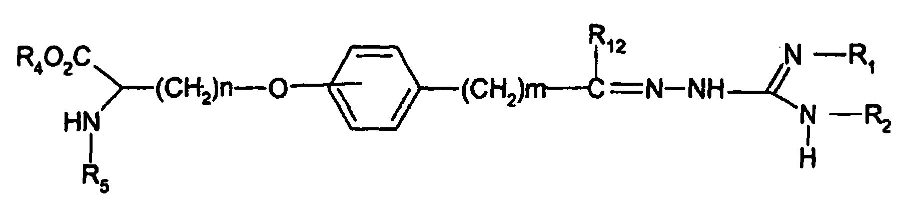 in der
R₅ ein Rest CO₂R₆ ist, R₆ -CH₂Ph ist, die genannten Verbindungen der Formel (I) in allen ihren möglichen enantiomeren, diastereoisomeren und/oder stereoisomeren Formen, allein oder in Mischung gemäß irgendeinem Verhältnis, wobei die an das Phenyl angrenzende Gruppe (Alkyl)iminoguanidin sich in Position para oder meta vom Sauerstoff befindet,
in der R₁ und R₂ ein Wasserstoffatom darstellen oder zusammen eine Gruppe -(CH₂)ₚ- bilden, in der p gleich 2 oder 3 ist, wobei der genannte Rest Alkylen oder die Gruppe -(CH₂)ₚ- nicht substituiert ist,
R₄ ein Wasserstoffatom oder eine Gruppe (C₁-C₆)-Alkyl darstellt,
R₁₂ ein Wasserstoffatom oder einen Rest (C₁-C₆) -Alkyl darstellt,
m ein Ganzes gleich 1 oder 2 ist,
n ein Ganzes gleich 2 ist,
sowie ihre physiologisch akzeptablen Salze.

2. Verbindung der Formel (I') wie in Anspruch 1 definiert, von der die Namen folgen:
- O-{4-[3-[(4,5-Dihydro-1H-imidazol-2-yl)-hydrazono]-butyl]-phenyl}-N-[(phenylmethoxy)-carbonyl]-homoserin,
- O-{4-[3-[(Aminoiminomethyl)-hydrazono]-butyl]-phenyl}-N-[(phenylmethoxy)-carbonyl]-homoserin,
- O-{4-[3-[(4,5-Dihydro-1H-imidazol-2-yl)-hydrazono]-propyl]-phenyl}-N-[(phenylmethoxy)-carbonyl]-homoserin,
- O-{4-[3-[(Aminoiminomethyl)-hydrazono]-propyl]-phenyl}-N-[(phenylmethoxy)-carbonyl]-homoserin,
- O-{4-[2-[(4,5-Dihydro-1H-imidazol-2-yl)-hydrazono]-propyl]-phenyl}-N-[(phenylmethoxy)-carbonyl]-homoserin,
- O-{4-[2-[(4,5-Dihydro-1H-imidazol-2-yl)-hydrazono]-propyl]-2-methoxyphenyl}-N-[(phenylmethoxy)-carbonyl]-homoserin,
- O-{4-[2-[(4,5-Dihydro-1H-imidazol-2-yl)-hydrazono]-propyl]-2-fluorphenyl}-N-[(phenylmethoxy)-carbonyl]-homoserin,
- O-{3-[3-[(Aminoiminomethyl)-hydrazono]-butyl]-phenyl}-N-[(phenylmethoxy)-carbonyl]-homoserin,
- O-{3-[3-[(4,5-Dihydro-1H-imidazol-2-yl)-hydrazono]-butyl]-phenyl}-N-[(phenylmethoxy)-carbonyl]-homoserin,
- O-{3-[2-[(4,5-Dihydro-1H-imidazol-2-yl)-hydrazono]-propyl]-phenyl}-N-[(phenylmethoxy)-carbonyl]-homoserin,
- O-{3-[3-[(Aminoiminomethyl)-hydrazono]-propyl]-phenyl}-N-[(phenylmethoxy)-carbonyl]-homoserin,
- O-{3-[3-[(4,5-Dihydro-1H-imidazol-2-yl)-hydrazono]-propyl]-phenyl}-N-[(phenylmethoxy)-carbonyl]-homoserin,
- O-{4-[(4,5-Dihydro-1H-imidazol-2-yl)-hydrazono]-methyl]-phenyl}-N-[(phenylmethoxy)-carbonyl]-homoserin,
sowie ihre physiologisch akzeptablen Salze.

3. Verfahren zur Herstellung der Verbindungen der Formel (I') wie in irgendeinem der Ansprüche 1 bis 2 definiert, das die Kupplung von zwei oder mehreren Fragmenten umfaßt, die durch Retrosynthese der Verbindungen der Formel (I') abgeleitet werden können.

4. Verfahren nach Anspruch 3, bei dem man ein Acyl oder Formyl der Formel (III) 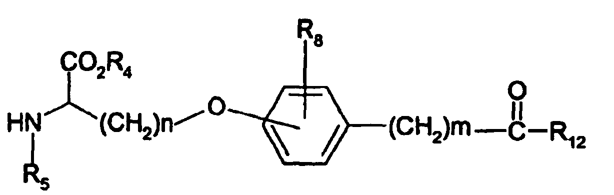 in der R₄, R₅, R₈, R₁₂, n und m wie in Anspruch 1 definiert sind und worin gegebenenfalls die funktionellen Gruppen in Form von Vorläufern oder in geschützter Form vorliegen, mit einem Aminoguanidin oder einem Derivat des Aminoguanidins der Formel (IV) 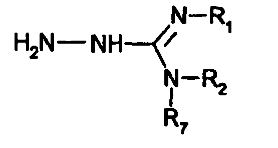 zur Reaktion bringt, in der R₁, R₂ und R₇ wie in Anspruch 1 definiert sind und worin gegebenenfalls die funktionellen Gruppen in Form von Vorläufern oder in geschützter Form vorliegen, wobei die genannten, gegebenenfalls in Form von Vorläufern oder in geschützter Form vorliegenden funktionellen Gruppen im weiteren Verlauf in Gruppen umgewandelt werden, die in den Verbindungen der Formel (I') dargestellt sind.

5. Als Arzneimittel eine Verbindung der Formel (I') und/oder ihre physiologisch akzeptablen Salze wie in irgendeinem der Ansprüche 1 bis 2 definiert.

6. Pharmazeutische Zusammensetzung, umfassend mindestens eine Verbindung der Formel (I') wie in irgendeinem der Ansprüche 1 bis 2 definiert und/oder ihre physiologisch akzeptablen Salze sowie einen oder mehrere übliche pharmazeutisch inerte Träger und gegebenenfalls einen oder mehrere Zusatzstoffe.

7. Als Arzneimittel mit einer antagonistischen Aktivität des Rezeptors von Vitronectin eine Verbindung der Formel (I') und/oder ihre physiologisch akzeptablen Salze wie in irgendeinem der Ansprüche 1 bis 2 definiert.

8. Als Arzneimittel mit einer Inhibitor-Aktivität der Knochenresorption oder für die Behandlung oder Vorbeugung von Osteoporose eine Verbindung der Formel (I') und/oder ihre physiologisch akzeptablen Salze wie in irgendeinem der Ansprüche 1 bis 2 definiert.

9. Als Arzneimittel mit einer Inhibitor-Aktivität des tumoralen Wachstums oder von Krebsmetastasen eine Verbindung der Formel (I') und/oder ihre physiologisch akzeptablen Salze wie in irgendeinem der Ansprüche 1 bis 2 definiert.

10. Als Arzneimittel mit einer anti-inflammatorischen Aktivität oder für die Behandlung oder Vorbeugung von cardiovasculären Störungen, Restenose, Arteriosklerose, Nephropathien oder Retinopathien eine Verbindung der Formel (I') und/oder ihre physiologisch akzeptablen Salze wie in irgendeinem der Ansprüche 1 bis 2 definiert.

11. Verwendung von Verbindungen der Formel (I') und/oder ihrer physiologisch akzeptablen Salze wie in irgendeinem der Ansprüche 1 bis 2 definiert zur Herstellung von Arzneimitteln, vorgesehen für die Vorbeugung oder Behandlung von Osteoporose.

12. Verwendung von Verbindungen der Formel (I') und/oder ihrer physiologisch akzeptablen Salze wie in irgendeinem der Ansprüche 1 bis 2 definiert zur Herstellung von Arzneimitteln, vorgesehen für die Inhibierung des tumoralen Wachstums oder von Krebsmetastasen.

13. Verwendung von Verbindungen der Formel (I') und/oder ihrer physiologisch akzeptablen Salze wie in irgendeinem der Ansprüche 1 bis 2 definiert zur Herstellung von Arzneimitteln, vorgesehen für die Vorbeugung oder Behandlung von cardiovasculären Störungen, Restenose, Arteriosklerose, Nephropathien oder Retinopathien.
